# EUROPEAN PATENT APPLICATION

(11) **EP 3 560 448 A1**
(43) Date of publication of application: **30.10.2019**
(21) Application number: 19170144.0
(22) Date of filing: 18.04.2019
(51) Int. Cl.: A61B 17/86

(54) **INTERFERENCE SCREW**

(30) Priority: 24.04.2018 PL 42533918
(71) Applicant: Mrugas, Robert, 03-264 Warszawa (PL); Radomskie Centrum Innowacji i Technologil sp. z o.o., 26-613 Radom (PL)
(72) Inventor: MRUGAS, Robert, 03-264 Warsaw (PL)
(74) Representative: Piotrowicz, Alicja

(57) **Abstract**

The subject of the invention is an interference screw comprising a screw body which includes a tip portion (1) having a front face (2), a main screw body with a screw thread on an external surface between a tip portion and an end portion (3) having a rear face (4). In addition, along the axis of the screw body is the internal passageway (5) and between protrusions (6) of the screw thread, in the thread roots (7), there are located through openings (8) that connect with the internal passageway. The solution is characterized in that it has
- the internal passageway (5) is located between the front face (2) of the tip portion (1) and the rear face (4) of the end portion (3) and it changes its size and cross section shape along its length, wherein the internal passageway (5) has a circular cross section in the tip portion (1) of the screw and the internal passageway has a polygon cross section in the main part of the screw body,
- in the main portion of the body, through openings (8) of the screw, provided within a wall of the main screw body, have a repetitive shape,
- the thread provided on the external surface of the main portion of the screw is a blunt interference thread, which task is to tighten the implants to the bone without causing damage
- roughness of the external surface of the screw is in the range from 25 to 35 µm, whereas the porosity of the outer surface of the screw is in the range from 0.01 mm to 2 mm
- it is coated with biocompatible and bactericidal materials.

## Description

This invention relates to an interference screw.

A prior art solution disclosed in document EP 2737865 A1 describes a bone screw that comprises: a tubular body having two ends - a first end and a second end, wherein the tubular body has a tubular wall defining a cavity and wherein a plurality of openings are situated extending through the tubular wall, and the screw also comprises an exterior thread on an exterior tubular surface of the tubular wall for anchoring into a bone, a head configured to engage with a driving tool to advance the bone screw in the bone by rotating the driving tool in a first direction, the head being also configured to connect to the tubular body at the first end. The head is also connectable to the tubular body in such a way that it is locked against disconnection from the tubular body when the driving tool is rotated in a second direction opposite to the first direction.

Another prior art solution is a bone anchoring element having a shaft for anchoring in a bone, as disclosed in a document EP 1749490 A1. At least one barb element is connected to the shaft. The barb element has a free end and a base connecting the barb element to the shaft. Moreover, the barb element has a first portion adjacent to the base and a second portion adjacent to the free end. The second portion is inclined relative to the first portion in a direction off said shaft.

Another invention relating to a screw, published in document US 6283973 B1, is a solution that comprises a screw body including a tip portion having a front face, an end portion having a rear face and being positioned at a certain distance from the tip portion, a threaded portion having a rounded thread extending between the tip portion and the end portion, and a passageway extending the length of the screw body from the front face to the rear face.

The solutions known so far exhibited adverse side effects, such as osteomalacia and ischaemia, missing innervation, as well as the implant sliding out.

The object of the present invention is to provide a screw which, unlike the existing solutions, allows transfer of torque to the entire length of the screw.

Another object of the invention is to provide a screw that allows improved flow of body fluids from the inside of the screw in a direction towards the bone, through specially designed openings on a lateral surface of the screw.

The subject matter of this patent application is an interference screw characterised in that:
- an internal passageway extends from a front face of a tip portion to a rear face of an end portion, the internal passageway changing its size and shape of cross section along its length, wherein the internal passageway has a circular cross section in the tip portion of the screw and the internal passageway has a polygon cross section in a main portion of the screw body,
- in the main portion of the screw body, openings extending through the screw are provided within a wall of the main portion of the screw body, the openings are of a repeatable shape,
- a thread provided on an external surface of the main portion of the screw is a blunt interference thread, which task is to press implants to a bone without causing damage,
- roughness of the external surface of the screw is within a range of 25 to 35 µm, whereas porosity of the screw is within a range of 0.01 mm to 2 mm,
- it is coated or uncoated with biocompatible and/or bactericidal materials.

Advantageously, the internal passageway has a hexagonal cross section from the end portion throughout the main portion of the screw body.

Advantageously, the openings extending through the screw are located circumferentially and spaced apart equidistantly from each other, and each lead of the thread has at least one opening.

Advantageously, openings extending through the screw, in the main portion of the screw body, are of essentially circular and/or oval, and/or polygonal, and/or irregular, and/or tear shape.

Advantageously, it is made from titanium powder for surgical implant applications. Advantageously, protrusions of the thread are rounded.

Advantageously, the end portion of the screw ends with a head.

Advantageously, roughness of the external surface is within a range of 25 to 35 µm. Advantageously, it is permanently coated with hydroxyapatite.

Advantageously, the bactericidal agents are metal alloys or oxides of zinc, copper, silver and nanosilver.

Advantageously, the screw is produced with use of a 3D laser printer.

Another invention is a method for the production of an interference screw characterised in that, on a fabrication bed, layers of titanium powder having thickness of 20 to 60 µm are laser sintered by the action of two coordinated laser beams having power of 100 to 400 Watts and scanning speed of 800 to 1600 mm/s and spot size of 40 to 190 µm. The sintering is performed in a chamber filled with protective gas atmosphere composed of argon with residual presence of oxygen not higher than 2%. Next, another layer of titanium powder is applied, which fuses with the previous layer. After the sintering process is completed, screws are cleaned from unsintered powder in argon atmosphere with residual oxygen content not higher than 2% and are disconnected from the fabrication bed with help of an electro-dowelling machine. Next, they are subjected to mechanical treatment and abrasive blasting treatment.

Advantageously, the powder used has grain size not larger than 63 µm. Advantageously, the abrasive blasting treatment is performed with glass beads of grain size in a range of 300 to 400 µm.

An embodiment of the invention has been presented in a drawing, in which the figures depict:
Fig. 1.0 - a perspective view of the interference screw as seen from the front face of the tip portion, in a first embodiment
Fig. 1.1 - a view of the interference screw from the front face of the tip portion, in a first embodiment
Fig. 1.2 - a longitudinal sectional view of the interference screw along the A-A line indicated in Fig. 1.1
Fig. 1.3- a transverse sectional view of the interference screw along the B-B line indicated in Fig. 1.2
Fig. 1.4- an enlarged sectional view of a fragment of the interference screw indicated by the letter C in Fig. 1.2
Fig. 2.0- a perspective view of the interference screw as seen from the front face of the tip portion, in a second embodiment
Fig. 2.1- a view of the interference screw from the front face of the tip portion, in a second embodiment
Fig. 2.2 - a longitudinal sectional view of the interference screw along the A-A line indicated in Fig. 2.1
Fig. 2.3- a transverse sectional view of the interference screw along the B-B line indicated in Fig. 2.2
Fig. 2.4- an enlarged sectional view of a fragment of the interference screw indicated by the letter C in Fig. 2.2
Fig. 3.0- a perspective view of the interference screw as seen from the front face of the tip portion, in a third embodiment
Fig. 3.1- a view of the interference screw from the front face of the tip portion, in a third embodiment
Fig. 3.2- a longitudinal sectional view of the interference screw along the A-A line indicated in Fig. 3.1
Fig. 3.3 - a transverse sectional view of the interference screw along the B-B line indicated in Fig. 3.2
Fig. 3.4- an enlarged sectional view of a fragment of the interference screw indicated by the letter C in Fig. 3.2
Fig. 4.0- a perspective view of the interference screw as seen from the front face of the tip portion, in a fourth embodiment
Fig. 4.1- a view of the interference screw from the front face of the tip portion, in a fourth embodiment
Fig. 4.2- a longitudinal sectional view of the interference screw along the A-A line indicated in Fig. 4.1
Fig. 4.3- a transverse sectional view of the interference screw along the B-B line indicated in Fig. 4.2
Fig. 4.4 - an enlarged sectional view of a fragment of the interference screw indicated by the letter C in Fig. 4.2
Fig. 5.0- a perspective view of the interference screw as seen from the front face of the tip portion, in a fifth embodiment
Fig. 5.1- a view of the interference screw from the front face of the tip portion, in a fifth embodiment
Fig. 5.2- a longitudinal sectional view of the interference screw along the A-A line indicated in Fig. 5.1
Fig. 5.3- a transverse sectional view of the interference screw along the B-B line indicated in Fig. 5.2
Fig. 5.4- an enlarged sectional view of a fragment of the interference screw indicated by the letter C in Fig. 5.2
Fig. 6.0- a perspective view of the interference screw as seen from the front face of the tip portion, in a sixth embodiment
Fig. 6.1- a view of the interference screw from the front face of the tip portion, in a sixth embodiment
Fig. 6.2- a longitudinal sectional view of the interference screw along the A-A line indicated in Fig. 6.1
Fig. 6.3- a transverse sectional view of the interference screw along the B-B line indicated in Fig. 6.2
Fig. 6.4- an enlarged sectional view of a fragment of the interference screw indicated by the letter C in Fig. 6.2
Fig. 7.0- a perspective view of the interference screw as seen from the front face of the tip portion, in a seventh embodiment
Fig. 7.1 - a view of the interference screw from the front face of the tip portion, in a seventh embodiment
Fig. 7.2- a longitudinal sectional view of the interference screw along the A-A line indicated in Fig. 7.1
Fig. 8.0 - a perspective view of the interference screw as seen from the front face of the tip portion, in a seventh embodiment
Fig. 8.1 - a view of the interference screw from the front face of the tip portion, in a seventh embodiment
Fig. 8.2 - a longitudinal sectional view of the interference screw along the A-A line indicated in Fig. 8.1

The objective set by the Inventors has been reached by means of developing an interference screw having an internal passageway extending throughout the entire length of the screw enabling employment of a driving tool and transfer of torque to the entire length of the screw. Thanks to the solution in accordance with invention it is now possible to place a driving tool inside the internal passageway starting from the rear face, along the main cylindrical portion having a thread on the external surface, through to the cylindrical inner opening of the tip portion. In the new solution, as the interference screw that secures an implant is being driven in, the screw are not damaged as a consequence of thread stripping. This provides work confidence to the surgeon performing the procedure.

The developed solution eliminates the difficulties related to adverse effects known in medicine, consisting in a hindered integration of the interference screw with the bone or with the implant. The construction of the subject element ensures improved flow of body fluids through the screw towards the bone. It turned out unexpectedly that this effect was reached as a result of providing a plurality of openings positioned on the surface of the main portion of the interference screw. The most favourable shape of the opening that enables quick tissue growth through the interference screw is a tear shape. Such shapes facilitate quicker innervation in the area of the surgical procedure and, by the same, quicker healing of the wound and quicker recovery of the patient. Moreover, an interference screw in accordance with the invention prevents osteomalacia.

Screws used in medicine and veterinary medicine so far had been manufactured by mechanical working. Valuable material used for the manufacture of such elements was being lost during the subsequent stages of production. The interference screw developed by the Inventors is made from implant grade titanium powder that is laser sintered during 3D printing. The material is medical implant grade titanium alloy Ti-6AI-4V AMTM F 136 as per ISO 5832:3 standard. As a result of 3D laser printer production, only such amount of material is used as it is necessary for the making of individual screws. Thus, the solution in accordance with the invention allows for decreasing the amount of valuable material used for the manufacturing of one interference screw, which translates into lower economic effect.

Thanks to a new shape of the screw with a blunt thread and also thanks to new shapes of openings on the lateral surface of the screw it was possible to improve adherence to the bone and to expedite the healing process by way of perfusion of body fluids. Owing to the roughness of the external surface of the screw it was possible to enlarge the contact surface and to facilitate nourishing an implant or bone tissue growth through a synthetic implant. Rate of bone growth was improved by means of coating the surface of the screw with hydroxyapatite, which forms an osteoinductive layer. Moreover, to prevent an inflammatory response in the body in the area of applying an implant screw, bactericidal agents are deposited on the surface of the implant screw.

Interference screws are used in veterinary medicine and human medicine in the upper and lower limb, in the shoulder and pelvic girdle, wherever natural or synthetic implants are grafted into tissues.

The screws developed by the Applicant may be manufactured in a variety of sizes and their length can be in a range of 8 mm to 40 mm, whereas the pitch can be changed every 1 mm. The diameter can also vary, from 3 mm to 12 mm. The screw size would depend on the anatomic conditions of the patient, whereas thread radius must be fitted to the screw diameter.

The interference screw comprises a screw body that includes a tip portion having a front face, which is "blunt" so as not to damage an implant during the surgical procedure. Along the axis, in the tip portion of the interference screw, there is a cylindrical internal passageway with a circular cross section. Between the tip portion and the end portion extends a main screw body having a screw thread on the external surface, which is a blunt thread. Thread protrusions are rounded and have no cutting capacity, which protects an implant from being cut.

In a main portion of the screw body, an internal passageway extends axially from the end portion through the main portion of the screw body toward the tip portion has a polygon cross section, preferably hexagonal cross section. This shape corresponds to the shape of a tip of a driving tool inserted into the passageway.

In the main portion of the screw body, between thread protrusions, in thread roots, located openings are located, which have a repeatable shape, and which connect with the internal passageway. The openings are located circumferentially and spaced apart equidistantly from each other, and each lead of the thread has at least one opening. The openings extending through the interference screw can have an essentially circular and/or oval, and/or polygonal, and/or irregular, and/or tear shape. However, the most advantageous shape for a through opening that facilitates quick adherence is a tear-shaped opening.

The interference screw has an end portion which has a rear face, which can be flat or can end with a head, depending on the need.

The screw in accordance with the invention is made from titanium powder for surgical implant applications providing roughness of the external surface of the screw within a range of 25 to 35 µm, whereas porosity of the screw is within a range of 0.01 mm to 2 mm.

The screw in accordance with the invention is made on a laser 3D printer. A method of manufacturing an interference screw in accordance with the invention consists in that on a fabrication bed, layers of titanium powder having thickness of 20 to 60 µm are laser sintered by the action of two coordinated laser beams having power of 100 to 400 Watts and scanning speed of 800 to 1600 mm/s and spot size of 40 to 190 µm. The sintering is performed in a chamber filled with protective gas atmosphere composed of argon with residual presence of oxygen not higher than 2%. Next, another layer of titanium powder is applied, which fuses with the previous layer. After the sintering process is completed, screws are cleaned from unsintered powder under argon atmosphere with residual oxygen content not higher than 2% and are disconnected from the fabrication bed with help of an electro-dowelling machine. Next, they are subjected to mechanical treatment and abrasive blasting treatment. Advantageously, the powder used has grain size not larger than 63 µm, and the abrasive blasting is performed with glass beads of grain size in the range of 300 to 400 µm.

The solution in accordance with the invention is represented by seven embodiments pertaining to a screw, where in each figure the same elements have the same numerical references.

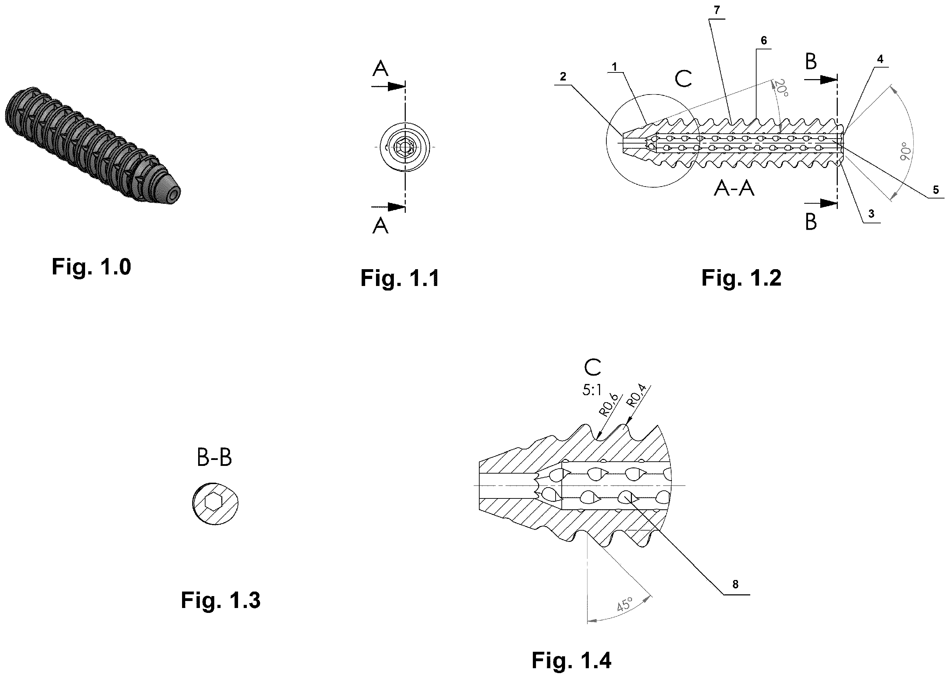

Figs. 1.0 - 1.4 show a first embodiment, of a 9x40 interference screw. This means that the outside diameter of the interference screw is 9 mm while its length is 40 mm. Along a main portion extends an internal passageway with a hexagonal cross section 4 mm in diameter, whereas in a tip portion an internal passageway has a circular cross section 1.85 mm in diameter. The angle of incline of the surface of the screw's tip portion is 20°. Extending through a wall of the main portion of the screw body equidistantly spaced tear-shaped openings, are provided. The screw thread provided on the surface has a lead angle of 45°, while thread crests end with a radius R = 0.4 and thread roots end with a radius R = 0.6. Thread lead is 2.7 mm, while thread height is 1.2 mm. In this embodiment, the interference screw has a rear face of the end portion located at the root of the last thread.

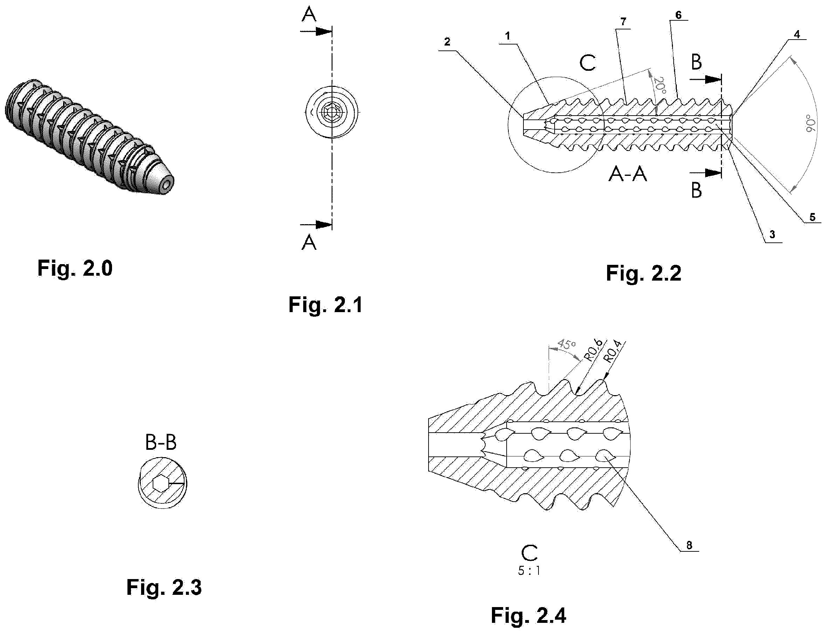

A second embodiment shown in Figs. 2.0 - 2.4 differs from the interference screw shown in Figs. 1.0 - 1.4 by the outside diameter of the screw, which is 1 mm larger, and the diameter of the internal passageway, which in this case is 0.5 mm larger.

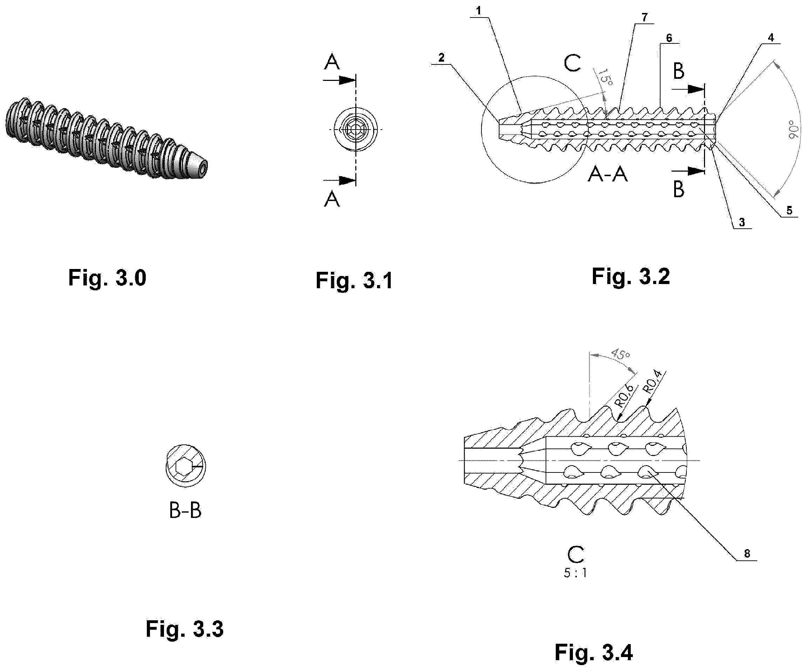

A solution shown in Figs. 3.0 - 3.4 is a third embodiment of the screw, which differs from the interference screw shown in Figs. 1.0 - 1.4 by the outside diameter of the screw, which is 1 mm smaller, and the diameter of the internal passageway, which in this case is 0.2 mm smaller. In this embodiment, the tip portion of the screw has the angle of incline of 15°.

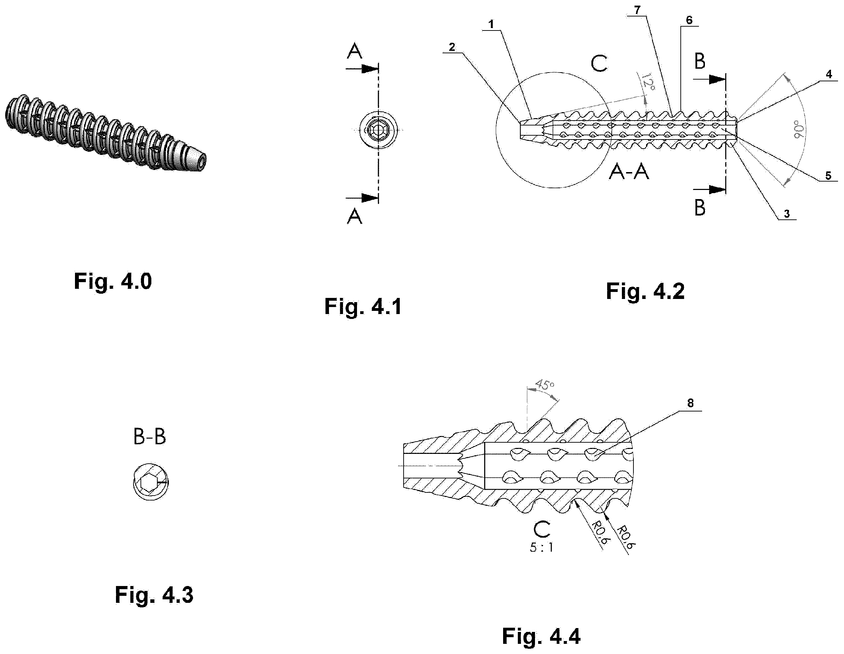

Another embodiment is shown in Figs. 4.0 - 4.4, and it is different from the interference screw shown in Figs. 1.0 - 1.4 by the outside diameter of the screw, which is 2 mm smaller, and the diameter of the internal passageway, which in this case is 0.4 mm smaller. In this embodiment, the tip portion of the screw has the angle of incline of 12°. Thread crests and thread roots end with a radius R = 0.6.

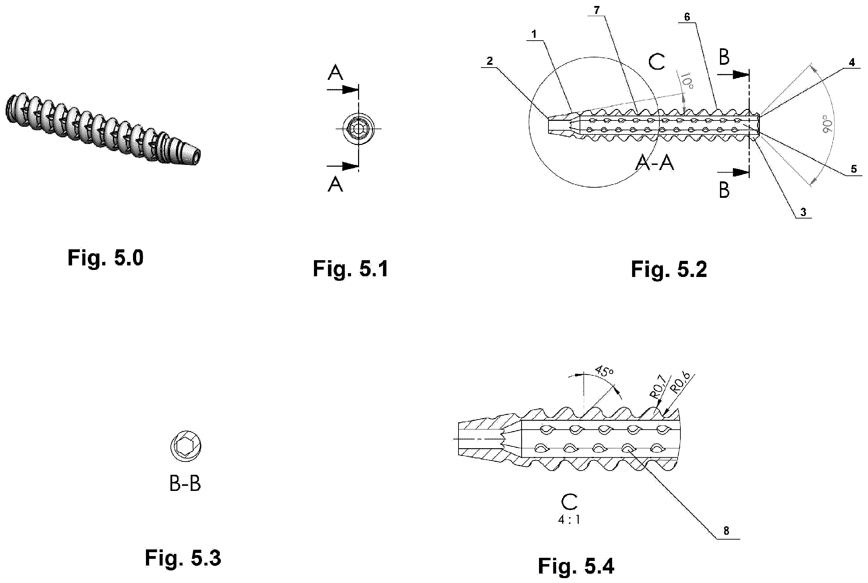

A fifth embodiment disclosed in Figs. 5.0 - 5.4 is different from the interference screw shown in Figs. 1.0 - 1.4 by the outside diameter of the screw, which is 3 mm smaller, and the diameter of the internal passageway, which in this case is 0.4 mm smaller. In this embodiment, the tip portion of the screw has the angle of incline of 10°. Thread crests end with a radius R = 0.7 and thread roots end with a radius R = 0.6, while thread height is 1 mm.

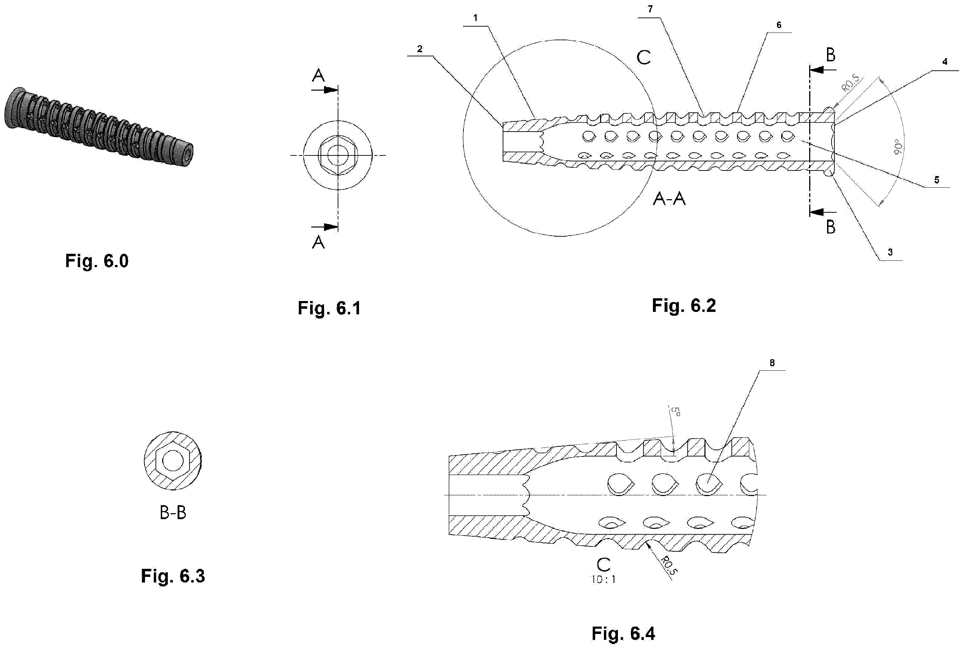

Figs. 6.0 - 6.4 show a sixth embodiment, of a 5x30 interference screw. Along a main portion extends an internal passageway with a hexagonal cross section of 5.2 mm in diameter, whereas in a tip portion an internal passageway has a circular cross section 1.85 mm in diameter. The angle of incline of the surface of the screw's tip portion is 5°. Extending through a wall of the main portion of the screw body, are provided equidistantly spaced tear-shaped openings. Thread roots end with a radius R = 0.5. Thread pitch is 2 mm, while thread height is 0.5 mm. In this embodiment, the interference screw has a rear face of the end portion in the form of a cylindrical flange terminated with a swelling located circumferentially.

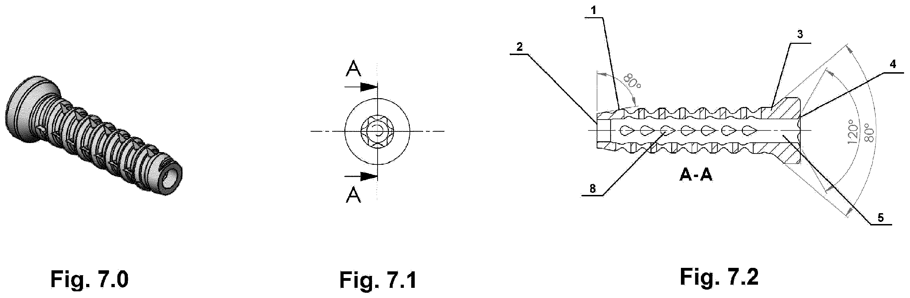

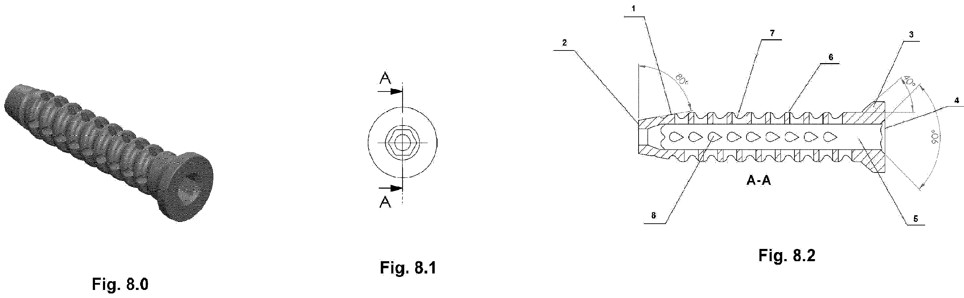

A seventh embodiment is shown in Figs. 7.0 - 7.2 and it discloses a veterinary interference screw. The end portion of the screw is in the form of a cylindrical flange that conically expands into a screw head which ending is cylindrical. An eighth embodiment disclosed in Figs. 8.0 - 8.2 is a variant of the screw shown in Figs. 7.0 - 7.2.

In a preferred embodiment the screw is made with a roughness of 25 µm, porosity of 0.3 mm, and is coated with bactericidal agents which include materials from the group of: metal alloys or their oxides of zinc, copper, silver, nanosilver.

## Claims

1. An interference screw comprising a screw body which includes a tip portion (1) having a front face (2), a main screw body provided with a screw thread on an external surface between a tip portion and an end portion, and an end portion (3) having a rear face (4), moreover, extending axially along the screw body there is an internal passageway (5), and between protrusions (6) of the screw thread, in thread roots (7), there are located through openings (8) that connect with the internal passageway, **characterised in that:**
- the internal passageway (5) extends from the front face (2) of the tip portion (1) to the rear face (4) of the end portion (3) and it changes its size and cross section along its length, wherein the internal passageway (5) has a circular cross section in the tip portion (2) of the screw and the internal passageway has a polygon cross section in a main portion of the screw body,
- in the main portion of the screw body, openings (8) extending through the screw, provided within a wall of the main screw body, have a repeatable shape,
- the thread provided on the external surface of the main portion of the screw is a blunt interference thread, which task is to press implants to a bone without causing damege,
- roughness of the external surface of the screw is within a range of 25 to 35 µm, whereas porosity of the screw is within a range of 0.01 mm to 2 mm,
- it is coated with biocompatible and bactericidal materials.

2. An interference screw in accordance with claim 1, **characterised in that** the internal passageway (5) has a hexagonal cross section from the end portion (3) throughout the main portion of the screw body.

3. An interference screw in accordance with claim 1, **characterised in that** the openings (8) extending through the screw are located circumferentially and spaced apart equidistantly from each other, and each lead of the thread has at least one opening.

4. An interference screw in accordance with claim 1, **characterised in that** the openings (8) extending through the screw have an essentially circular and/or oval, and/or polygonal, and/or irregular, and/or tear shape.

5. An interference screw in accordance with claim 1, **characterised in that** it is made from titanium powder for surgical implant applications.

6. An interference screw in accordance with claim 1, **characterised in that** the protrusions (6) of the thread are rounded.

7. An interference screw in accordance with claim 1, **characterised in that** the end portion (3) of the screw terminates with a head (9).

8. An interference screw in accordance with claim 1, **characterised in that** roughness of the external surface is within a range of 25 to 35 µm.

9. An interference screw in accordance with claim 1, **characterised in that** it is permanently coated with hydroxyapatite.

10. An interference screw in accordance with claim 1, **characterised in that** the bactericidal agents are metal alloys or oxides of zinc, copper, silver and nanosilver.

11. An interference screw in accordance with claim 1, **characterised in that** it is produced with use of a 3D laser printer.
